# EUROPEAN PATENT SPECIFICATION

(11) **EP 1 456 361 B1**
(45) Date of publication and mention of the grant of the patent: **29.02.2012**
(21) Application number: 02781173.6
(22) Date of filing: 04.12.2002
(51) Int. Cl.: C12N 9/28, C12N 9/54, C07K 1/30, C07K 1/36

(54) **PROCESS FOR HARVESTING CRYSTALLINE PARTICLES FROM FERMENTATION BROTH**
VERFAHREN ZUR SAMMLUNG VON KRISTALLINEN PARTIKELN AUS FERMENTATIONSBRÜHE
PRODUCTION DE CRISTAUX A PARTIR DE BOUILLON DE FERMENTATION

(30) Priority: 11.12.2001 DK 200101847
(43) Date of publication of application: 15.09.2004
(73) Proprietor: Novozymes A/S, 2880 Bagsvaerd (DK)
(72) Inventor: NIELSEN, Benny, DK-4532 Gislinge (DK); RANCKE-MADSEN, Anders, DK-2920 Charlottenlund (DK); JOERGENSEN, Martin, Troen, DK-4000 Roskilde (DK)
(74) Representative: Kofoed, Gertrud Sonne
(86) International application number: PCT/DK2002/000821
(87) International publication number: WO 2003/050274

(56) References cited:
- EP-A- 0 574 050
- WO-A-95/01989
- WO-A-96/38469
- FR-A- 2 679 235
- US-A- 4 659 667
- BAJPAI P ET AL: "CLARIFICATION OF BACTERIAL BROTH CONTAINING HIGH ALPHA-AMYLASE ACTIVITY" BIOTECHNOLOGY TECHNIQUES, vol. 4, no. 4, 1990, pages 227-232, XP008008107 ISSN: 0951-208X

## Description

### TECHNICAL FIELD

The present invention relates to a simple and effective method for obtaining a crystalline alpha-amylase or protease suspension from a fermentation broth.

### BACKGROUND ART

EP 0 574 050 discloses that highly pure fermentation products with a hydrophobic character can be obtained from a large scale fermentation broth in one single step by liquid phase separation by flocculation the cell-solids, addition of an amphiphilic detergent and a suitable salt.

### SUMMARY OF THE INVENTION

It has surprisingly been found that a simple and effective method for producing a crystalline alpha-amylase or protease suspension from a fermentation broth may be produced by (a) treating the fermentation broth with calcium chloride and one or more polymers; and (b) separating the fermentation broth, by use of a separation equipment, into a biomass fraction, a crystalline alpha-amylase or protease fraction, and a supernatant fraction.

### DETAILED DISCLOSURE OF THE INVENTION

The present invention provides a simple and effective method for producing a crystalline alpha-amylase or protease suspension from a fermentation broth.

The method of the invention may be applied to an untreated fermentation broth or to a fermentation broth that has first been subjected to, e.g., a pH adjustment, a temperature adjustment, and/or a water dilution.

### Metabolites of interest

The metabolite of interest according to the invention may be an alpha-amylase or protease.

Proteases: Suitable proteases include those of animal, vegetable or microbial origin. Microbial origin is preferred. Chemically modified or protein engineered mutants are included. The proteases may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 91/00345, WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 195, 206, 218, 222, 224, 235 and 274.

Amylases: Suitable amylases (alpha and/or beta) include those of bacterial or fungal origin. Chemically modified or protein engineered mutants are included. Amylases include, for example, alpha-amylases obtained from *Bacillus,* e.g. a special strain of *B*. *licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

### Fermentations

The fermentation broth according to the invention may be obtained from any microorganism of any genus known in the art.

In a preferred embodiment, the metabolite of interest may be obtained from a bacterial or a fungal source. For example, the metabolite of interest may be obtained from a gram positive bacterium such as a *Bacillus* strain, *e.g., Bacillus alkalophilus, Bacillus amyloliquefaciens, Bacillus brevis, Bacillus circulans, Bacillus coagulans, Bacillus lautus, Bacillus lentus, Bacillus clausii, Bacillus licheniformis, Bacillus megaterium, Bacillus stearothermophilus, Bacillus subtilis,* or *Bacillus thuringiensis;* or a *Streptomyces* strain, *e.g., Streptomyces lividans* or *Streptomyces murinus;* or from a gram negative bacterium, *e.g., E. coli* or *Pseudomonas* sp.

The metabolite of interest may be obtained from a fungal source, e.g. from a yeast strain such as a *Candida, Kluyveromyces, Pichia, Saccharomyces, Schizosaccharomyces,* or *Yarroviria* strain, e.g., *Saccharomyces carlsbergensis, Saccharomyces cerevisiae, Saccharomyces diastaticus, Saccharomyces douglasii, Saccharomyces kluvveri, Saccharomyces norbensis* or *Saccharomyces oviformis* strain.

In a preferred embodiment the metabolite of interest may be obtained from a filamentous fungal strain such as an *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia, Tolypocladium,* or *Trichoderma* strain, in particular the metabolite of interest may be obtained from an *Aspergillus aculeatus, Aspergillus awamori, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Trichoderma harzianum, Trichoderma koningii, Trichoderma longibrachiatum, Trichoderma reesei,* or *Trichoderma viride* strain.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

For purposes of the present invention, the term "obtained from" as used herein in connection with a given source shall mean that the metabolite of interest is produced by the source or by a cell in which a gene from the source has been inserted.

The microbial strain may be fermented by any method known in the art. The fermentation medium may be a complex medium comprising complex nitrogen and/or carbon sources, such as soybean meal, cotton seed meal, corn steep liquor, yeast extract, casein hydrolysate, molasses, and the like. The fermentation medium may be a chemically defined media, e.g. as defined in WO 98/37179.

The fermentation may be performed as a fed-batch, a repeated fed-batch or a continuous fermentation process.

### Coagulants and flocculants

According to the disclosure a useful coagulant may be a salt such as one selected from the group consisting of the group I metal salts, the group II metal salts, and the group III metal salts, or mixtures thereof; in particular Ca-, Mg- and Al-salts. Preferred salts are ammonium, phosphate, sulfate, carbonate, and citrate salts. Halogenide salts, formiates and acetates may also be applicable, especially chloride salts such as calcium chloride.

Useful salt concentrations will be in the range of 0.1-40% (w/w); preferably in the range of 0.2-20% (w/w); more preferably in the range of 0.3-6% (w/w).

According to the disclosure useful coagulant is a short chained polymer, in particular a cationic polymer with a molecular weight in the range of from 20 Daltons to 500000 Daltons, such as a tertiary or a quaternary polyamine, e.g. C521 obtainable from Cytec Industries, Poly-DADMAC's (Di-allyl Dimethyl Ammonium Chlorid), e.g. C591, or Aluminimu sources such as polyaluminumchlorohydrate: Al2(OH)5Cl, e.g. GC850 obtainable from Gulbrandsen.

Useful short chained polymer concentrations will typically be in the range of 0.1-25% (w/w); preferably in the range of 0.2-20% (w/w); more preferably in the range of 0.3-15% (w/w).

It will often be an advantage to add more than one coagulant, e.g. a salt and one or more short chained polymers.

According to the invention a useful flocculant may be an inorganic and/or organic polymer which may be cationic, anionic or non-ionic.

A useful cationic polymer is a polyamine, and a useful anionic polymer is a polyacrylamid.

Useful polymer concentrations will be in the range of 0.01-1.0% (w/w); preferably in the range of 0.05-0.5% (w/w).

### Separation equipment

According to the invention a useful separation equipment is any design of a two-phase centrifuge, especially a continuous sludge decharging centrifuge, a decanter or a cyclone.

It may in some cases be an advantage to add a flocculatant, in particular an anionic polymer, to the separation equipment in order to avoid biomass in the crystalline and/or amorphous suspension (see Example 1).

### Crystalline and/or amorphous suspension

It is possible to coagulate and/or flocculate the metabolite fermentation broth so that the crystalline metabolites are in the right separation zone and thus can be separated in e.g. a centrifuge process into a biomass fraction (with a very low metabolite concentration), the crystalline metabolite fraction (with a high metabolite concentration) and the supernatant fraction (with a very low metabolite concentration). The coagulation and/or the flocculation has the effect that the crystalline metabolites are not incorporated in the flocks, so after the biomass has been separated the crystalline metabolites may be further concentrated in order to achieve the wanted yield.

The suspension achieved according to the invention may be further purified in a variety of ways such as by using grinding, sieving, drying, filtration, centrifugation, recrystallisation, chromatographic methods, adsorption processes and/or two-phase extraction

The invention is further illustrated in the following examples which are not intended to be in any way limiting to the scope of the invention as claimed.

### EXAMPLE 1

### Harvest of protease crystals from fermentation broth

A fermentation broth containing a mutated subtilisin protease obtained from a *Bacillus sp.* disclosed in WO 91/00345 was subjected to the method of the invention.

The protease fermentation broth was run in production scale based on the following coagulation/flocculation recipe:

### Temperature: 12°C

| | |
|---|---|
| Dilution: | 2 times with water |
| CaCl₂ (36% w/w): | 4.7% |
| C521 (17% w/w): | 12.0% |
| GC-850 (20% w/w): | 5.0% |
| NaOH (3% w/w): | pH 7.5 |

The trial was made on an entire protease batch using one Westfalia SB60 centrifuge. The feed contained 17-20% wet sludge volume.

The centrifuge used was equipped with a nozzle bowl and a standardized sludge flow of 4 m3/hr.

The trial ran 21 hours where the feed flow was step-wise increased from 7.5 m3/h to 9.5 m3/hr.

In order to avoid biomass in the centrate, an anionic polymer was added at feed flows above 7.5 m3/hr.

The anionic polymer solution used was 0.15% (w/w) (polyacrylamid).

The percent wet crystal volume was measured in the centrate during the trial and used as yield indicator.

Table 1 shows the results.

Instead of using a Westfalia SB60 centrifuge a test was run using two Alfa Laval NX418 decanters as a first step extraction.

The test shows that the centrifuge process is superior to the decanter process in terms of capacity and yield.

**TABLE 1:**

| | Decanter | Centrifuge | Interval between Empting the bowl |
|---|---|---|---|
| Flow | 2000 1/h | 7500 1/h | 15 min. |
| Anionic polymer | 400 1/h | 0.0 1/h | |
| % crystal | 0.4 - 0.6% | 0.6 - 1.0% | |
| Flow | | 8000 1/h | 15 min. |
| Anionic polymer | | 50 1/h | |
| at, crystal | | 0.6 - 12% | |
| Flow | | 8500 1/h | 15 min. |
| Anionic polymer | | 100 1/h | |
| % crystal | | 0.6 - 1.5% | |
| Flow | | 9000 1/h | 15 min. |
| Anionic polymer | | 200 1/h | |
| % crystal | | 0.7 - 1.5% | |
| Flow | | 9500 1/h | 20 min. |
| Anionic polymer | | 250 1/h | |
| % crystal | | 0.7 - 1.5% | |
| % sludqe | | 0.1 - 0.3% | |

### EXAMPLE 2

### Harvest of alpha-amylase crystals from fermentation broth

An alpha-amylase obtained from a Bacillus sp. strain disclosed in SEQ ID NO: 2 of WO 95/26397 with a double deletion (D183*+G184*) as described in WO 96/23873, was fermented.

The alpha-amylase fermentation broth was taken out from production harvest tank and tests were done in lab based on the following flocculation recipe:

### Temperature: 12°C

| | |
|---|---|
| Dilution: | 2 times with water |
| CaCl₂ (36% w/w): | 4.7% |
| C521 (17% w/w): | 12.0% |
| GC-850 (20% w/w): | 5.0% |
| NaOH (3% w/w): | pH 7.5 |

It was possible to flocculate the alpha-amylase fermentation broth, so that the enzyme crystals were in the right separation zone (limits particles) and thus be separated in a centrifuge process.

The activity in the supernatant of the flocculated broth showed that only 0.8% of the alpha-amylase enzyme was in the solution.

## Claims

1. A method for producing a crystalline alpha-amylase or protease suspension from a fermentation broth comprising
(a) treating the fermentation broth with calcium chloride and one or more polymers; and
(b) separating the fermentation broth, by use of a separation equipment, into a biomass fraction, a crystalline alpha-amylase or protease fraction, and a supernatant fraction.

2. The method according to claim 1, wherein the polymer has a molecular weight in the range of from 20 Daltons to 500000 Daltons.

3. The method according to claim 1, wherein the polymer is a cationic or an anionic polymer.

4. The method according to claim 3, wherein the polymer is a polyamine or a polyacrylamid.

5. The method according to claim 1, wherein the separation equipment is selected from the group consisting of a centrifuge, a decanter, and a cyclone.

6. The method according to claim 5, wherein the centrifuge is a continuous sludge de-charging centrifuge.

## Patentansprüche

1. Verfahren zum Herstellen einer kristallinen alpha-Amylase- oder Proteasesuspension aus einer Fermentationsbrühe, umfassend
(a) Behandeln der Fermentationsbrühe mit Calciumchlorid und einem oder mehreren Polymeren; und
(b) Abtrennen der Fermentationsbrühe durch Verwendung einer Abtrennungsapparatur in eine Biomassefraktion, eine kristalline Alpha-Amylase- oder Proteasefraktion und eine Üb erstandfrakti on.

2. Verfahren nach Anspruch 1, wobei das Polymer ein Molekulargewicht in dem Bereich von 20 Dalton bis 500.000 Dalton aufweist.

3. Verfahren nach Anspruch 1, wobei das Polymer ein kationisches oder ein anionisches Polymer ist.

4. Verfahren nach Anspruch 3, wobei das Polymer ein Polyamin oder ein Polyacrylamid ist.

5. Verfahren nach Anspruch 1, wobei die Abtrennungsapparatur ausgewählt ist aus der Gruppe, bestehend aus einer Zentrifuge, einem Dekanter und einem Fliehkraftabscheider (Zyklon).

6. Verfahren nach Anspruch 5, wobei die Zentrifuge eine kontinuierlich Absatz-entziehende Zentrifuge ist.

## Revendications

1. Procédé de production d'une suspension cristalline d'alpha-amylase ou de protéase à partir d'un bouillon de fermentation comprenant :
(a) traiter le bouillon de fermentation avec du chlorure de calcium et un ou plusieurs polymères ; et
(b) séparer le bouillon de fermentation en utilisant un matériel de séparation en une fraction de biomasse, une fraction cristalline d'alpha-amylase ou de protéase et une fraction de surnageant.

2. Procédé selon la revendication 1, dans lequel le polymère a un poids moléculaire compris entre 20 daltons et 500000 Daltons.

3. Procédé selon la revendication 1, dans lequel le polymère est un polymère cationique ou anionique.

4. Procédé selon la revendication 3, dans lequel le polymère est une polyamine ou un polyacrylamide.

5. Procédé selon la revendication 1, dans lequel le matériel de séparation est choisi dans le groupe consistant en une centrifugeuse, un décanteur et un cyclone.

6. Procédé selon la revendication 5, dans lequel la centrifugeuse est une centrifugeuse à déchargement de boues continu.
